# EUROPÄISCHE PATENTSCHRIFT

(11) **EP 1 716 803 B1**
(45) Veröffentlichungstag und Bekanntmachung des Hinweises auf die Patenterteilung: **21.05.2008**
(21) Anmeldenummer: 06004680.2
(22) Anmeldetag: 08.03.2006
(51) Int. Cl.: B08B 9/02, A61B 1/12, A61B 19/00, A61B 1/012

(54) **Verfahren und Vorrichtung zur Überprüfung der Durchgängigkeit von langgestreckten Hohlkörpern, insbesondere von Endoskopkanälen**
Method and device for verifying the patency of elongated hollow bodies, in particular endoscope channels
Procédé et dispositif pour vérifier le passage d'un corps creux allongé, en particulier des canaux d'un endoscope

(30) Priorität: 24.03.2005 DE 102005014429
(43) Veröffentlichungstag der Anmeldung: 02.11.2006
(73) Patentinhaber: BHT Hygienetechnik GmbH, 86368 Gersthofen (DE)
(72) Erfinder: Annecke, Karl Heinz, 64625 Bensheim (DE)
(74) Vertreter: von Bülow, Tam

(56) Entgegenhaltungen:
- EP-A1- 0 711 529
- EP-A1- 1 502 538
- DE-A1- 10 208 035
- GB-A- 2 275 341

## Beschreibung

Die Erfindung bezieht sich auf ein Verfahren zur Überprüfung der Durchgängigkeit von langgestreckten Hohlkörpern, insbesondere von Endoskopkanälen, gemäß dem Oberbegriff des Patentanspruches 1 sowie auf eine Vorrichtung zur Durchführung des Verfahrens gemäß dem Oberbegriff des Patentanspruches 7.

Verfahren und Vorrichtung dieser Art sind aus der DE 102 08 035 A1 bekannt. Zur Überprüfung der Durchgängigkeit von Endoskopkanälen wird dort vorgeschlagen, ein bestimmtes Volumen einer Flüssigkeit mit einem vorbestimmten Druck durch den zu überprüfenden Endoskopkanal zu drücken und die Zeitdauer für diesen Vorgang zu messen. Liegt die gemessene Zeitdauer innerhalb vorgegebener Grenzen, so wird der Endoskopkanal als einwandfrei durchgängig bewertet.

Aus der DE 44 40 363 A1 ist es ebenfalls bekannt, zur Überprüfung der Durchgängigkeit von Endoskopkanälen diese einzeln oder in Gruppen nacheinander zu prüfen, indem den Kanälen Fluid aus einer Fluidquelle zugeführt wird und die Durchströmung des Kanales gemessen wird. Dies kann durch Messen des Fluidvolumens pro Zeiteinheit erfolgen oder dadurch, daß dem Kanal Fluid aus einer unter Druck stehenden Fluidquelle zugeführt wird und der Druckabfall pro Zeiteinheit gemessen wird. Der Meßwert wird dann in einer Steuereinheit mit gerätespezifisch vorgegebenen Daten verglichen. Bei Übereinstimmung der Meßwerte mit für einen speziellen Instrumententyp bekannten oder vorab ermittelten Daten kann das der Prüfung unterworfene Instrument als ein Instrument dieses Instrumententyps identifiziert und als durchspülbar erkannt werden.

Die US 4,721,123 beschreibt ein ähnliches Verfahren, bei dem eine Flüssigkeit unter Druck durch einen Kanal geleitet und der Druckverlauf gemessen wird. Die Meßwerte werden einem Computer zugeführt, dessen Programm die Druck/Volumen-Kurve mit einer Standard-Druck/Volumen-Kurve für neue Katheter desselben Modells vergleicht.

Ähnliche Verfahren und Vorrichtungen zur Überprüfung langer dünner Kanäle und insbesondere Endoskopkanäle sind auch aus der EP 0 711 529 A1, DE 44 34 114 A1, US 5,551,462, DE 36 01 395 A1, EP 0 709 056 A1, US 5,738,824, GB 2 275 341 A, EP 0 072 257 A2 und US 2004173008 A1 bekannt.

Schließlich ist in der älteren deutschen Patentanmeldung DE 103 52 198 ein Verfahren zur Analyse von Endoskopkanälen beschrieben, bei dem Flüssigkeit durch den zu überprüfenden Kanal geleitet und ein mit der Durchlässigkeit des Kanales verknüpfter Meßwert ermittelt wird, wobei der Meßwert in Zuordnung zu einem identifizierten Kanal gespeichert wird, der Vorgang mit Speichern der Meßwerte in Zuordnung zur Reihenfolge der Messungen mehrfach wiederholt wird und nach bekannten mathematischen Methoden eine Trendanalyse der Meßwerte durchgeführt wird. Aus dieser Trendanalyse läßt sich ermitteln, ob einzelne Kanäle eines Endoskopes durch Ablagerungen langsam verengt werden und abschätzen, nach wievielen zukünftigen Einsätzen das Endoskop voraussichtlich unbrauchbar wird.

Ein Verfahren gemäß dem Oberbegriff des Anspruches 1 und eine Vorrichtung zur Durchführung dieses Verfahrens sind aus EP 1 502 538 bekannt.

Der Durchfluß eines Fluides durch einen Kanal hängt neben den Eigenschaften des Kanales, wie Querschnitt, Oberfläche der Kanalwände, Form und Verlauf des Kanals, auch stark von der Viskosität des Fluides ab, die ihrerseits wiederum stark von den physikalischen Eigenschaften des Fluides und von der Temperatur abhängt. Bei Flüssigkeiten, die zum Reinigen von Endoskopen verwendet werden, hängt die Viskosität auch von der Zugabe von Waschmitteln, Weichmachern etc. ab. So ändert sich die Viskosität von Wasser bei einer Temperatursteigerung von 25°C auf 80°C ungefähr um den Faktor 2, d.h. das Wasser wird doppelt so "flüssig" wie es bei niedrigen Temperaturen ist. Dies hat natürlich einen starken Einfluß auf die Menge der Flüssigkeit, die bei einem vorgegebenen Druck innerhalb einer vorgegebenen Zeiteinheit durch ein langes enges Lumen mit konstantem Querschnitt, wie z.B. durch einen Endoskopkanal, fließt.

Für präzise Messungen muß daher der temperatur- und waschmittelabhängige Einfluß der Viskosität berücksichtigt werden. In der Praxis erfolgte dies dadurch, daß Temperatur und Waschmittelkonzentration bei den jeweiligen Messungen konstant gehalten wurden. Dies hat jedoch eine aufwendige Regelung der Temperatur und der Zugabe von Waschmitteln, Weichmachern usw. zur Folge.

Eine mögliche Lösung des Problems unterschiedlicher Viskositäten wäre es, die Viskosität bei der aktuellen Messung zu erfassen, beispielsweise indem man die Flüssigkeit durch einen Referenzkanal mit bekanntem Querschnitt, bekannter Länge und ohne irgendwelche Verschmutzungen oder Verengungen leitet, den Durchfluß (durchströmtes Volumen pro Zeiteinheit) mißt und die Meßwerte der zu überprüfenden Kanäle entsprechend der vom Referenzkanal ermittelten Viskosität umrechnet. Auch dieses Verfahren wäre aufwendig, da stets ein einwandfreier Referenzkanal bereitgehalten werden müßte und nicht sichergestellt ist, daß dieser nach längerem Gebrauch nicht auch seine Eigenschaften ändert.

Aufgabe der Erfindung ist es daher, Verfahren und Vorrichtung der eingangs genannten Art dahingehend zu verbessern, daß auch bei unterschiedlichen Viskositäten des bei der Messung verwendeten Fluides stets ein korrektes Meßergebnis erhalten wird.

Diese Aufgabe wird durch das Verfahren des Patentanspruches 1 und durch die Vorrichtung des Patentanspruches 7 gelöst.

Die Erfindung geht davon aus, daß bei einem Endoskop mehr als ein Kanal gleichzeitig überprüft wird. Weiter geht die Erfindung davon aus, daß mit sehr hoher Wahrscheinlichkeit nicht alle Kanäle gleichzeitig durch Verschmutzungen so verengt sind, daß der Durchfluß in ähnlicher Größenordnung reduziert wird. Basierend auf diesen Annahmen wird es als Grundprinzip der Erfindung angesehen, den Durchfluß durch einen Kanal auf den Gesamtdurchfluß durch alle Kanäle zu beziehen. Unter Durchfluß wird im folgenden stets ein durch einen Kanal fließendes Volumen eines Fluides pro Zeiteinheit verstanden, wobei als weiterer Parameter der Druck des Fluides oder der zeitliche Druckverlauf berücksichtigt werden.

Für ein jeweiliges Endoskop wird im einwandfreien Zustand aller Kanäle, also insbesondere im Neuzustand, eine Messung durchgeführt und das Ergebnis für jeden einzelnen Kanal gespeichert. Das gespeicherte Ergebnis ist Durchfluß durch den einzelnen Kanal bezogen auf den Gesamtdurchfluß durch alle Kanäle oder bezogen auf den Mittelwert des Durchflusses durch alle Kanäle. Man erhält im Ergebnis also eine Größe als Referenzwert, die angibt, welchen Anteil jeder einzelne Kanal am Gesamtdurchfluß oder am Mittelwert des Gesamtdurchflusses hat.

Bei einer aktuellen Messung an einem gebrauchten und gereinigten Endoskop werden dieselben Meßwerte ermittelt, also wieder Durchfluß des einzelnen Kanales bezogen auf Gesamtdurchfluß oder Mittelwert des Gesamtdurchflusses. Der aktuelle Wert wird dann mit dem Referenzwert verglichen, woraus erkannt werden kann, ob ein Kanal seine Eigenschaften verändert hat, also gegenüber dem Ursprungszustand verengt ist oder auch undicht ist, wobei sich letzteres durch einen höheren Durchfluß bemerkbar macht.

Dadurch, daß die Messung auf den aktuellen Gesamtdurchfluß bezogen wird, wird jeglicher Einfluß der Viskosität eliminiert. Ändert sich nämlich die Viskosität, so verändert sich auch der Gesamtdurchfluß und bei einwandfreien Kanälen auch der Durchfluß durch den einzelnen Kanal im selben Verhältnis.

Ein weiterer Vorteil der Erfindung besteht auch darin, daß sonstige externe Einflüsse auf das Endoskop, die sich gleichartig auf alle Kanäle auswirken, wie z.B. andere Lagerung oder Anhebung des Distalendes während der Messung, keinen Einfluß auf das Meßergebnis haben.

Im folgenden wird die Erfindung anhand eines Ausführungsbeispieles im Zusammenhang mit der Zeichnung ausführlicher erläutert. Es zeigt:
- Fig. 1: eine Prinzipskizze einer Vorrichtung zum Bestimmen der Durchgängigkeit von Endoskopkanälen;
- Fig. 2: eine Prinzipskizze ähnlich Fig. 1 bei einer anderen Ausführungsform; und
- Fig. 3: ein Prinzipschaltbild einer Auswerteeinheit, die bei der Vorrichtung der Fig. 1 oder 2 verwendet wird.

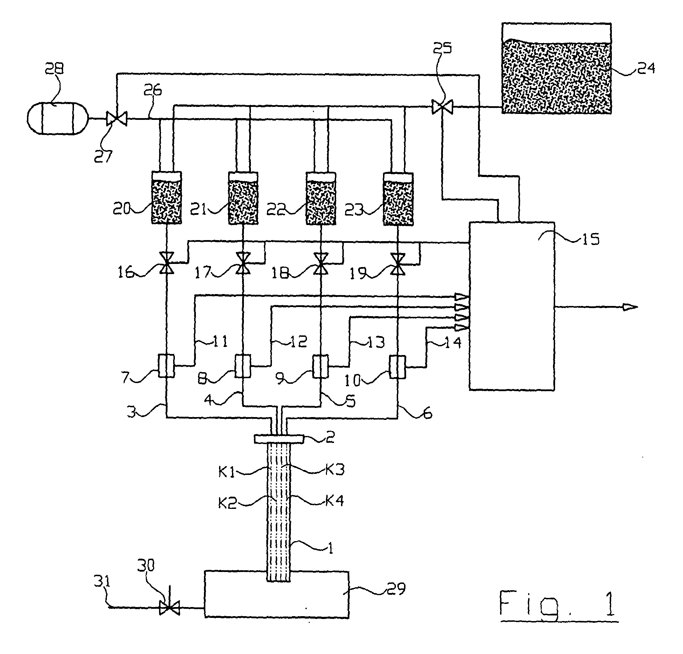

In Fig. 1 ist schematisch ein Endoskop 1 mit vier Kanälen K1, K2, K3, K4 dargestellt, die auf Durchgängigkeit überprüft werden sollen. Alle Kanäle K1 bis K4 sind hier über eine Kupplungsvorrichtung 2 an Fluidleitungen 3, 4, 5 bzw. 6 angeschlossen, über die jedem einzelnen Kanal K1 bis K4 individuell ein Fluid zugeführt werden kann. In jeder Fluidleitung 3 bis 6 ist ein Durchflußmesser 7, 8, 9, 10 vorhanden, der die durch die einzelne Leitung 3 bis 6 fließende Fluidmenge (Volumen) oder direkt den entsprechenden Durchfluß (Volumen pro Zeiteinheit) mißt und als elektrisches Signal über eine Leitung 11, 12, 13 bzw. 14 an eine Steuer- und Auswerteeinheit 15 liefert.

Im konkret dargestellten Ausführungsbeispiel der Fig. 1 ist jeder Fluidleitung 3 bis 6 ein elektrisch steuerbares Ventil 16, 17, 18 bzw. 19 zugeordnet, das von der Steuerund Auswerteeinheit 15 betätigbar ist, um Beginn und Ende der Messung festzulegen. Weiter ist hier jeder der Fluidleitung 3 bis 6 ein Vorratsbehälter 20, 21, 22, 23 zugeordnet, der ein vorbestimmtes Volumen des Fluides enthält. Damit ist nicht nur das Volumen des Fluides, das durch den individuellen Kanal fließen muß, bekannt sondern auch das Gesamtvolumen aller Vorratsbehälter 20 bis 23. Die Vorratsbehälter 20 bis 23 können aus einem gemeinsamen Reservoir 24 über ein Ventil 25 befüllt werden, was von der Steuer- und Auswerteeinheit 15 gesteuert werden kann.

Zusätzlich sind hier alle Vorratsbehälter 20 bis 23 über eine gemeinsame Leitung 26 und ein Ventil 27 an eine Druckluftquelle 28 angeschlossen, mit der das in den Vorratsbehältern 20 bis 23 befindliche Fluid mit einem vorbestimmten Druck beaufschlagt werden kann.

Da bei diesem Ausführungsbeispiel das Volumen, das durch die einzelnen Kanäle K1 bis K4 fließt, bekannt ist, können die Durchflußmesser 7 bis 10 auch als einfache Druckschalter ausgebildet sein, mit denen durch einen Druckabfall festgestellt werden kann, ob bzw. wann der einzelne Vorratsbehälter vollständig entleert ist, sein komplettes Volumen also durch den zugeordneten Kanal K1 bis K4 geflossen ist. Anstelle von Druckschaltern können auch Schwimmerschalter in den Vorratsbehältern 20 bis 23 verwendet werden oder sonstige bekannte Pegeldetektoren, die feststellen, wann der Fluidpegel in den Vorratsbehältern 20 bis 23 unter einen vorbestimmten Wert gesunken ist.

Der Begriff "Durchflußmesser" ist demgemäß im weitesten Sinne zu verstehen und umfaßt jegliche Meßeinrichtung, über die direkt oder indirekt ein Durchfluß (Volumen pro Zeiteinheit) ermittelt werden kann. Da bei den hier verwendeten Fluiden das spezifische Gewicht bekannt ist, sind auch Meßeinrichtungen verwendbar, die direkt oder indirekt eine Masse pro Zeiteinheit erfassen.

Fig. 1 zeigt noch, daß ein distales Ende des Endoskops 1 in einen Behälter 29 mündet, der beispielsweise auch der Sumpf einer Reinigungs- und Desinfektionsmaschine sein kann und der über ein Ventil 30 entleert wird, beispielsweise in einen Abwasserkanal 31.

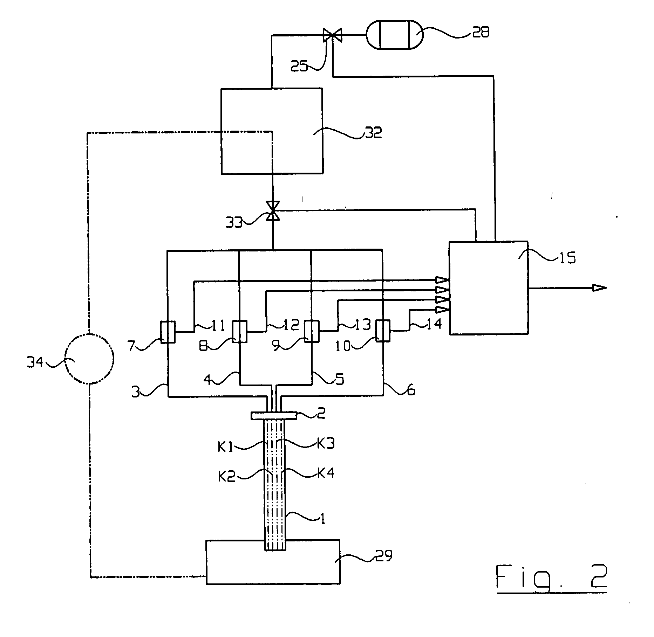

Im Ausführungsbeispiel der Fig. 2 werden alle Fluidleitungen 3 bis 6 aus einer gemeinsamen Fluidquelle 32 gespeist, die über ein Ventil 33 mit den Fluidleitungen 3 bis 6 verbindbar ist. Die Durchflußmesser 7 bis 10 messen dann für jede einzelne Leitung 3 bis 6 bzw. jeden einzelnen Kanal K1 bis K4 das in einer vorgegebenen Meßzeit durchfließende Volumen und übermitteln den entsprechenden Meßwert über die Leitungen 11 bis 14 an die Steuer- und Auswerteeinheit 15. Die Meßzeit beginnt mit dem Öffnen des Ventils 33 und endet mit dessen Schließen.

Zur Einstellung des Fluiddruckes ist auch hier eine Druckluftquelle 28 vorgesehen, die über das Ventil 25 aktivierbar ist und das Fluid in der Fluidquelle 32 mit einem vorgegebenen Druck beaufschlagt.

Statt einer Druckluftquelle kann selbstverständlich auch eine andere Form der Druckregelung verwendet werden, beispielsweise eine geregelte Pumpe, die das Fluid der Fluidquelle in die Leitungen 3 bis 6 pumpt. Hierbei kann es sich bei einer Reinigungs- und Desinfektionsmaschine auch um eine Umwälzpumpe 34 handeln, die das Fluid aus dem Sumpf 29 der Maschine unmittelbar in die Leitungen 3 bis 6 pumpt, wobei dann die Fluidquelle 32 und die Druckluftquelle 28 fortgelassen werden können sowie auch das Ventil 33, da eine Messung dann dadurch eingeleitet wird, daß die ständig anliegenden Signale der Durchflußmesser 7 bis 10 für ein vorgegebenes Zeitintervall eingelesen werden.

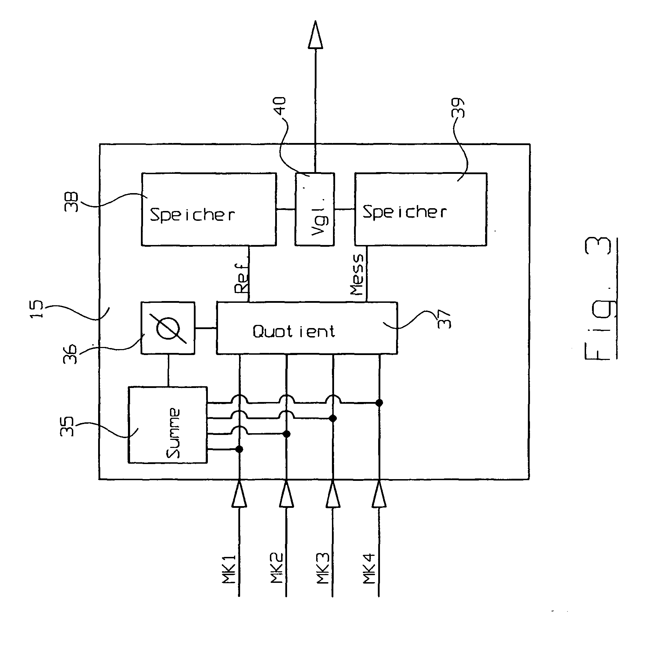

Fig. 3 zeigt ein Blockschaltbild eines Teiles der Steuerund Auswerteeinheit 15, die hier vier Eingänge für die Meßwerte MK1 bis MK4 hat, an denen die Ausgangssignale der Durchflußmesser 7 bis 10 für die Kanäle K1 bis K4 liegen. Es sei hier angenommen, daß die Meßwerte in der Maßeinheit Volumen pro Zeiteinheit (also z.B. cm³/s) vorliegen. Von diesen Meßwerten wird in einem Summierer 35 die Summe gebildet und in einem Rechenbaustein 36 der Durchschnittswert, d.h. Summe geteilt durch Anzahl der Kanäle. In einem weiteren Rechenbaustein 37 wird der Quotient aus den einzelnen Meßwerten MK1 bis MK4 geteilt durch den Mittelwert oder geteilt durch die Summe gebildet. Bei einer anfänglichen Messung eines einwandfreien Endoskopes werden die Werte aus dem Rechenbaustein 37 für jeden einzelnen Kanal in einem Speicher 38 als Referenzwerte abgelegt. Bei nachfolgenden Messungen an demselben aber gebrauchten Endoskop werden dieselben Werte in derselben Weise gebildet und bedarfsweise in einem Speicher 39 zwischengespeichert. Schließlich werden die im Speicher 38 gespeicherten Referenzwerte und die aktuellen Meßwerte in einem Vergleicher 40 verglichen. Liegen die Abweichungen außerhalb eines vorgegebenen Toleranzbandes, so meldet der Vergleicher 40 dies in Form eines Ausgangssignales, das im Ergebnis anzeigt, daß ein oder mehrere Kanäle einen Anteil am Gesamtdurchfluß bzw. am Mittelwert des Gesamtdurchflusses haben, der von dem entsprechenden Wert eines einwandfreien Endoskopes abweicht.

Dem Fachmann ist klar, daß die in Fig. 3 dargestellten einzelnen Rechenbausteine durch einen einzigen Mikroprozessor und einen einzigen daran angeschlossenen Speicher realisiert werden können. Die sonstigen erforderlichen Bausteine, wie Taktgeber und Zeitsteuerung, für die einzelnen Ventile sind der Übersichtlichkeit halber fortgelassen.

Nachfolgend wird im Zusammenhang mit Tabelle 1 das Verfahren nach der Erfindung genauer beschrieben. Es sei angenommen, daß ein Endoskop die vier Kanäle K1 bis K4 habe, die alle den gleichen Durchmesser haben und somit auch denselben Durchfluß aufweisen müssen. An einem einwandfreien Endoskop wird nun der Durchfluß bestimmt, der der Einfachkeit halber den Wert 100 betrage. Die Summe ergibt sich somit zu 400 und der Mittelwert zu 100. Bezieht man den Durchfluß auf den Mittelwert von 100, so beträgt er für jeden Kanal 100 %. Dies wird für jeden Kanal als Referenzwert 1 gespeichert. Alternativ kann man den Durchfluß auch auf die Summe beziehen, womit sich ein zweiter Referenzwert von je 25 % ergibt.

Beim zweiten Fall der Tabelle 1 sei die Viskosität um 10 % höher. Man sieht, daß die Abweichungen von den Referenzwerten 1 und 2 trotz verändertem Durchfluß wiederum den Wert von 0 % ergeben. Gleiches gilt für verringerte Viskosität im dritten Falle der Tabelle 1.

Im vierten Fall der Tabelle 1 sei angenommen, daß der Kanal K2 verengt ist. Die Abweichung des auf den Mittelwert bezogenen Durchflusses liegt hier bei -7,69 %. Da der Anteil der übrigen einwandfreien Kanäle bezogen auf den Mittelwert sich vergrößert, haben diese dann eine positivere Abweichung von 2,56 %. Als Kriterium für einen verengten Kanal kann man somit bei dieser Konstellation auch das Vorzeichen der Abweichung verwenden, wobei man aber in der Praxis besser auch den Zahlenwert der Abweichung berücksichtigt.

Im fünften Fall der Tabelle 1 sei angenommen, daß der Kanal K1 stark verengt ist und nur noch 50 betrage. Dementsprechend ergibt sich eine starke Abweichung gegenüber dem Referenzwert 1 mit negativem Vorzeichen, währen die übrigen Kanäle K2 bis K4 eine relativ große positive Abweichung vom Referenzwert 1 haben, nämlich 14,29 %

Nach einer Weiterbildung der Erfindung kann man dann folgendermaßen vorgehen:
Bei der weiteren Auswertung wird der stark verengte Kanal K1 unberücksichtigt gelassen und es werden nur für die "guten" Kanäle K2 bis K4 der Mittelwert des Durchflusses, die Abweichung von diesem Mittelwert und die Abweichung vom Referenzwert errechnet, wobei sich die Abweichung vom Referenzwert 1 dann wiederum zu 0 ergibt. Damit ist eindeutig geklärt, daß nur der Kanal K1 defekt ist, während die übrigen Kanäle K2 bis K4 einwandfrei durchgängig sind.

Im siebten Fall sei schließlich angenommen, daß, wie im vierten Fall, wiederum der Kanal K2 verengt ist und gleichzeitig aber das Fluid eine 10 % höhere Viskosität hat. Man sieht, daß auch in diesem Fall der Einfluß der Viskosität eliminiert ist, da die Abweichung vom Referenzwert 1 identisch wie beim vierten Fall ist.

**Tabelle 1**

| | **K1** | **K2** | **K3** | **K4** | **Summe** | **Mittelwert** |
|---|---|---|---|---|---|---|
| **1.) Gleiche Kanäle (NEU)** | | | | | | |
| Durchfluß(cm3/s) (Referenz) | 100 | 100 | 100 | 100 | 400 | 100 |
| % von Mittelwert (Referenz 1) | 100,00% | 100,00% | 100,00% | 100,00% | | |
| % von Summe (Referenz 2) | 25,00% | 25,00% | 25,00% | 25,00% | | |
| %Abw von Mittelwert | 0 | 0 | 0 | 0 | | |
| | | | | | | |

| **2.) 10% höhere Viskosität** | | | | | | |
|---|---|---|---|---|---|---|
| Durchfluß(cm3/s) IST | 110 | 110 | 110 | 110 | 440 | 110 |
| % von Mittelwert | 100,00% | 100,00% | 100,00% | 100,00% | | |
| %Abw von Referenz 1 | 0,00% | 0,00% | 0,00% | 0,00% | | |
| % von Summe | 25,00% | 25,00% | 25,00% | 25,00% | | |
| % Abw. von Refrenz 2 | 0,00% | 0,00% | 0,00% | 0,00% | | |
| | | | | | | |

| **3.) 10% geringere Viskosität** | | | | | | |
|---|---|---|---|---|---|---|
| Durchfluß(cm3/s) IST | 90 | 90 | 90 | 90 | 360 | 90 |
| % von Mittelwert | 100,00% | 100,00% | 100,00% | 100,00% | | |
| %Abw von Referenz 1 | 0,00% | 0,00% | 0,00% | 0,00% | | |
| % von Summe | 25,00% | 25,00% | 25,00% | 25,00% | | |
| % Abw. von Refrenz 2 | 0,00% | 0,00% | 0,00% | 0,00% | | |
| | | | | | | |

| **4.) 1 Kanal verengt** | | | | | | |
|---|---|---|---|---|---|---|
| Durchfluß(cm3/s) IST | 100 | **90** | 100 | 100 | 390 | 97,5 |
| % von Mittelwert | 102,56% | **92,31%** | 102,56% | 102,56% | | |
| %Abw von Referenz 1 | 2,56% | **-7,69%** | 2,56% | 2,56% | | |
| | | | | | | |

| **5.) Mittelwert aus K1-K4** | verengt | | | | | |
|---|---|---|---|---|---|---|
| Durchfluß(cm3/s) IST | 50 | 100 | 100 | 100 | 350 | 87,5 |
| % von Mittelwert | 57,14% | 114,29% | 114,29% | 114,29% | | |
| %Abw von Referenz 1 | -42,86% | 14,29% | 14,29% | 14,29% | | |
| | | | | | | |

| **6.) Mittelwert aus K2-K4** | verengt | | | | | |
|---|---|---|---|---|---|---|
| Durchfluß(cm3/s) IST | **50** | 100 | 100 | 100 | 300 | 100 |
| % von Mittelwert | **50,00%** | 100,00% | 100,00% | 100,00% | 100,00% | |
| %Abw von Referenz 1 | **NB** | 0,00% | 0,00% | 0,00% | | |
| | | | | | | |

| **7.) 10% höhere Viskosität** | | verengt | | | | |
|---|---|---|---|---|---|---|
| Durchfluß(cm3/s) IST | 110 | 99 | 110 | 110 | 429 | 107,25 |
| % von Mittelwert | 102,56% | 92,31% | 102,56% | 102,56% | | |
| %Abw von Referenz 1 | 2,56% | -7,69% | 2,56% | 2,56% | | |

Für die Praxis ist es meist nicht nötig, bei einem defekten Endoskop den oder die defekten Kanäle zu identifizieren. Defekt in diesem Sinne bedeutet, daß der Kanal verengt oder undicht ist, sein relativer Durchfluß bezogen auf den Gesamtdurchfluß oder den Durchschnitt des Durchflusses aller Kanäle vom entsprechenden Referenzwert abweicht. Gleichwohl ist es, wie die in der Tabelle 1 gezeigten Beispiele zeigen, bei vielen Fallkonstellationen mit der Erfindung auch möglich, den oder die defekten Kanäle zu identifizieren. Verengte Kanäle werden dadurch erkannt, daß die Differenz zwischen einem Sollwert (Referenzwert), der bei einwandfreiem Endoskop ermittelt wurde, und dem aktuellen Meßwert negativ ist, während diese Differenz für die intakten Kanäle dann positiv wird, da ihr Anteil am Gesamtdurchfluß vergrößert wird, wenn ein oder mehrere Kanäle verengt sind. Mit diesen Kriterien lassen sich somit verengte Kanäle sogar identifizieren. In ähnlicher Weise läßt sich auch ein undichter Kanal dadurch identifizieren, daß für ihn dann die Differenz positiv wird, während sie für alle intakten Kanäle negativ ist. Zweckmäßiger ist es natürlich, nicht nur das Vorzeichen dieser Differenz auszuwerten, sondern auch den Absolutbetrag und für diesen einen Grenzwert vorzugeben, ab welchem ein Endoskopkanal als nicht mehr einwandfrei durchlässig definiert wird, da in der Praxis auch einwandfrei gereinigte Endoskopkanäle ihre Durchlässigkeit verändern. Sofern diese Veränderungen innerhalb einer bestimmten Bandbreite gleich verlaufen, kann dies mit der vorliegenden Erfindung nicht erkannt werden, da der gleiche Effekt auch durch Änderung der Viskosität hervorgerufen werden kann.

Nach einer Weiterbildung der Erfindung läßt sich das Verfahren dahingehend verfeinern, daß für die Bildung des Gesamtdurchflusses oder die Durchschnittsbildung nicht alle Kanäle herangezogen werden sondern nur ausgewählte Kanäle. Die Auswahl erfolgt vorzugsweise so, daß Einzelkanäle mit sehr starken Abweichungen, die größer als ein vorgegebener Grenzwert sind, vor der Durchschnittsbildung eliminiert werden.

Damit läßt sich der eliminierte Kanal aussondern und genauer überprüfen, ob die verbleibenden Kanäle einwandfrei durchgängig sind. Dieses Verfahrens kann auch iterativ für unterschiedliche Kombinationen von Kanälen durchgeführt werden, um so defekte Kanäle besser identifizieren zu können.

In der Praxis hat sich herausgestellt, daß undichte Kanäle häufig deshalb signalisiert werden, weil die entsprechende Fluidleitung nicht korrekt an den jeweiligen Kanal angeschlossen ist, was primär ein Problem der verwendeten Kupplung ist. Wird ein undichter Kanal festgestellt, so wird in der Praxis die Kupplung überprüft und der Meßvorgang noch einmal wiederholt. Wird dagegen ein verengter Kanal festgestellt, so wird die Reinigung des Endoskopes als fehlerhaft verworfen. In diesen Fällen kann das Endoskop erneut gereinigt und überprüft werden oder ausgesondert zur Reparatur gesandt werden.

## Patentansprüche

1. Verfahren zur Überprüfung der Durchgängigkeit von langgestreckten Kanälen, insbesondere Endoskopkanälen, bei dem je ein auf die durch die Kanäle strömende Fluidmenge pro Zeiteinheit bezogener Meßwert ermittelt und mit einem Referenzwert verglichen wird,
**dadurch gekennzeichnet,**
**daß** der jeweilige Referenzwert eines Kanales die durch diesen Kanal fließende Fluidmenge pro Zeiteinheit bezogen auf die durch mehrere Kanäle fließende Fluidmenge pro Zeiteinheit bei einwandfrei durchgängigen Kanälen ist und
**daß** der jeweilige Meßwert in analoger Weise die durch den zu überprüfenden Kanal fließende Fluidmenge pro Zeiteinheit bezogen auf die durch mehrere Kanäle fließende Fluidmenge pro Zeiteinheit ist.

2. Verfahren nach Anspruch 1, **dadurch gekennzeichnet, daß** der Referenzwert und der Meßwert jeweils auf die durch alle zu überprüfenden Kanäle fließende Fluidmenge pro Zeiteinheit bezogen ist.

3. Verfahren nach Anspruch 1 oder 2, **dadurch gekennzeichnet,**
**daß** der Referenzwert und der Meßwert jeweils auf den Durchschnitt der durch mehrere oder alle zu überprüfenden Kanäle fließenden Fluidmenge bezogen sind.

4. Verfahren nach Anspruch 3, **dadurch gekennzeichnet, daß** ein Kanal als nicht ausreichend durchgängig bestimmt wird, wenn der Absolutwert der Differenz zwischen dem Meßwert und dem Referenzwert dieses Kanales von einem vorgegebenen Wert abweicht.

5. Verfahren nach Anspruch 4, **dadurch gekennzeichnet, daß** der Meßwert und der Referenzwert gespeichert werden.

6. Verfahren nach Anspruch 4, **dadurch gekennzeichnet, daß** der Meßwert und der Referenzwert nur auf solche Kanäle bezogen werden, bei denen der Absolutwert der Differenz zwischen dem Meßwert und dem Referenzwert kleiner als ein vorgegebener Wert ist.

7. Vorrichtung zur Durchführung des Verfahrens nach einem oder mehreren der Ansprüche 1 bis 6,
mit einer Fluidquelle (24, 32), die über Leitungen (3, 4, 5, 6) mit zu überprüfenden Kanälen (K1, K2, K3, K4) verbindbar ist,
mit Meßeinrichtungen (7, 8, 9, 10), die einen auf die durch den jeweiligen Kanal (K1...K4) fließende Fluidmenge pro Zeiteinheit bezogenen Meßwert ermitteln, und
mit einer Auswerteeinheit (15), die den jeweiligen Meßwert mit einem in einem Speicher (38) gespeicherten Referenzwert vergleicht,
**dadurch gekennzeichnet,**
**daß** die Auswerteeinheit (15) einen Summierer (35) aufweist, dessen Eingänge mit den Meßeinrichtungen (7, 8, 9, 10) verbunden sind,
**daß** ein Dividierer (37) vorgesehen ist, dessen einer Eingang mit den Meßeinrichtungen (7, 8, 9, 10) und dessen anderer Eingang mit dem Ausgang des Summierers (35) verbunden ist, und
**daß** der Ausgang des Dividierers (37) mit einem Vergleicher (40) verbunden ist, dessen anderer Eingang mit dem Speicher (38) verbunden ist, in dem der Referenzwert gespeichert ist.

8. Vorrichtung nach Anspruch 7, **dadurch gekennzeichnet, daß** zwischen dem Summierer (35) und dem Dividierer (37) ein Rechenbaustein (36) zwischengeschaltet ist, der einen Mittelwert des Ausgangssignals des Summierers bildet und dem Dividierer (37) zuführt.

9. Vorrichtung nach Anspruch 8, **dadurch gekennzeichnet, daß** der Rechenbaustein (36) das arithmetische Mittel bildet.

10. Vorrichtung nach einem der Ansprüche 7 bis 9, **dadurch gekennzeichnet,**
**daß** zwischen dem Dividierer (37) und dem Vergleicher (40) ein Speicher (39) geschaltet ist, in dem die Meßwerte gespeichert werden.

## Claims

1. Method for checking the fluid passing characteristics of elongated channels, in particular of endoscope channels, where a measured value referred to the fluid amount flowing through each of the channels per time unit is measured and is compared with a reference value,
**characterized in that**
each reference value of a channel is defined as the fluid amount flowing through this channel per time unit referred to the fluid amount flowing through several channels with correct unobstructed channels and
**in that** each measured value is defined in an analog way by the fluid amount flowing through the channel to be checked per time unit referred to the fluid amount flowing through several channels per time unit.

2. Method according to claim 1, **characterized in that** the reference value and the measured value both are referred to the fluid amount per time unit flowing through all channels to be checked.

3. Method according to claim 1 or 2, **characterized in that** the reference value and the measured value both are referred to the average fluid amount flowing through several or all channels to be checked.

4. Method according to claim 3, **characterized in that** a channel is defined as not sufficient free, if the absolute value of the difference between the measured value and the reference value of this channel differs from a predefined value.

5. Method according to claim 4, **characterized in that** the measured value and the reference value are stored.

6. Method according to claim 4, **characterized in that** the measured value and the reference value are referred only to such channels, where the absolute value of the difference between the measured value and the reference value is smaller than a predefined value.

7. Apparatus for performing the method according to one or more of claims 1 to 6,
comprising a fluid source (24, 32), which is connectable via pipes (3, 4, 5, 6) with channels (K1, K2, K3, K4) to be checked,
with measuring devices (7, 8, 9, 10), which detect a measured value of a fluid amount per time unit flowing through the individual channel (K1...K4) and with a evaluation unit (15), which compares each measured value with a reference value stored in a memory (38),
**characterized in that** the evaluation unit (15) comprises a summing device (35), whose inputs are connected with the measuring devices (7, 8, 9, 10), **in that** a dividing device (37) is provided, whose one input is connected with the measuring devices (7, 8, 9, 10) and whose other input is connected with the output of the summing device (35), and
**in that** the output of the dividing device (37) is connected with a comparing device (40), whose other input is connected with the memory (38), where the reference value is stored.

8. Apparatus according to claim 7, **characterized in that** a calculating device (36) is connected between the summing device (35) and the dividing device (37), the calculating device is forming an average value of the output signals of the summing device and is feeding it to the dividing device (37).

9. Apparatus according to claim 8, **characterized in that** the calculation device (36) is forming the arithmetical average value.

10. Apparatus according to one of claims 7 to 9, **characterized in that** a memory (39) is connected between the dividing device (37) and the comparing device (40), wherein the measured values are stored in that memory (39).

## Revendications

1. Procédé pour vérifier le passage dans des canaux allongés, en particulier des canaux d'endoscope, dans lequel on détermine par mesure une quantité de fluide s'écoulant dans les canaux par unité de temps, et on la compare à une valeur de référence,
**caractérisé en ce que** la valeur de référence respective d'un canal est la quantité de fluide s'écoulant dans ce canal par unité de temps évaluée en référence à la quantité de fluide s'écoulant dans plusieurs canaux par unité de temps lorsque les canaux peuvent être parcourus librement, et
**en ce que** la valeur mesurée respective est, de manière analogue, la quantité de fluide s'écoulant par le canal à vérifier par unité de temps évaluée en référence à la quantité de fluide s'écoulant dans plusieurs canaux par unité de temps.

2. Procédé selon la revendication 1, **caractérisé en ce que** la valeur de référence et la valeur mesurée sont évaluées en référence respectivement à la quantité de fluide s'écoulant dans tous les canaux à vérifier par unité de temps.

3. Procédé selon l'une des revendications 1 ou 2, **caractérisé en ce que** la valeur de référence et la valeur mesurée sont comparées respectivement à la moyenne de la quantité de fluide s'écoulant dans plusieurs ou dans tous les canaux à vérifier.

4. Procédé selon la revendication 3, **caractérisé en ce qu'**un canal est déterminé comme n'assurant pas un écoulement suffisant lorsque la valeur absolue de la différence entre la valeur mesurée et la valeur de référence de ce canal dépasse une valeur prédéterminée.

5. Procédé selon la revendication 4, **caractérisé en ce que** la valeur mesurée et la valeur de référence sont enregistrées.

6. Procédé selon la revendication 4, **caractérisé en ce que** la valeur mesurée et la valeur de référence ne sont évaluées qu'en référence aux canaux dans lesquels la valeur absolue de la différence entre la valeur mesurée et la valeur de référence est inférieure à une valeur prédéterminée.

7. Dispositif pour la mise en oeuvre du procédé selon une ou plusieurs des revendications 1 à 6, comportant
une source de fluide (24, 32) qui peut être reliée par des conduites (3, 4, 5, 6) aux canaux à vérifier (K1, K2, K3, K4),
des dispositifs de mesure (7, 8, 9, 10) qui déterminent une valeur mesurée par unité de temps de la quantité de fluide s'écoulant par le canal respectif (K1...K4), et
une unité d'analyse (15) qui compare la valeur mesurée respective à une valeur de référence enregistrée dans une mémoire (38),
**caractérisé en ce que** l'unité d'analyse (15) présente un additionneur (35) dont les entrées sont reliées aux dispositifs de mesure (7, 8, 9, 10),
**en ce qu'**un diviseur (37) est prévu, dont une entrée est reliée aux dispositifs de mesure (7, 8, 9, 10) et dont l'autre entrée est reliée à la sortie de l'additionneur (35), et
**en ce que** la sortie du diviseur (37) est reliée à un comparateur (40) dont l'autre entrée est reliée à la mémoire (38) dans laquelle est enregistrée la valeur de référence.

8. Dispositif selon la revendication 7, **caractérisé en ce que**, entre l'additionneur (35) et le diviseur (37) est intercalé un bloc de calcul (36) qui forme une valeur moyenne du signal de sortie de l'additionneur et alimente le diviseur (37).

9. Dispositif selon la revendication 8, **caractérisé en ce que** le bloc de calcul (36) réalise une moyenne arithmétique.

10. Dispositif selon l'une quelconque des revendications 7 à 9, **caractérisé en ce que**, entre le diviseur (37) et le comparateur (40) est insérée une mémoire (39) dans laquelle sont enregistrées les valeurs mesurées.
